(19) Europäisches Patentamt
European Patent Office
Office européen des brevets

(11) Veröffentlichungsnummer: **0 154 035**
**B1**

(12) # EUROPÄISCHE PATENTSCHRIFT

(45) Veröffentlichungstag der Patentschrift: **25.07.90**

(21) Anmeldenummer: **84116411.4**

(22) Anmeldetag: **27.12.84**

(51) Int. Cl.$^5$: **C 12 N 9/02**, C 12 P 17/12, C 07 D 455/02, C 07 D 455/06

(54) **S-Tetrahydroprotoberberinoxidase, Verfahren zu deren Herstellung ind ihre Verwendung.**

(30) Priorität: **24.02.84 DE 3406774**

(43) Veröffentlichungstag der Anmeldung:
**11.09.85 Patentblatt 85/37**

(45) Bekanntmachung des Hinweises auf die Patenterteilung:
**25.07.90 Patentblatt 90/30**

(84) Benannte Vertragsstaaten:
**AT CH DE LI SE**

(56) Entgegenhaltungen:
**EP-A-0 028 959**
**DE-A-2 552 630**

**CHEMICAL ABSTRACTS, Band 86, Nr. 15, 11. April 1977, Seite 536, Zusammenfassung Nr. 106845v, Columbus, Ohio, US; H.-C. CHIANG et al.: "Protoberberine alkaloids. Synthesis of 3,9-dihydroxy-2,10-dimethoxytetrahydroprotoberberine and 3,11-dihydroxy-2,10-dimethoxytetrahydropseudoberberine", & TAI-WAN YAO HSUEH TSA CHIH 1975, 27(1-2), 90-7**

(73) Patentinhaber: **Consortium für elektrochemische Industrie GmbH**
**Zielstattstrasse 20**
**D-8000 München 70 (DE)**

(72) Erfinder: **Amann, Manfred**
**Wörnbrunn 3**
**D-8022 Grünwald (DE)**
Erfinder: **Nagakura, Naotaka, Prof. Dr.**
**4-3-17-304 Tsurukabuto Nada-Ku**
**657 Kobe (JP)**
Erfinder: **Zenk, Meinhart H. Prof. Dr.**
**Pfeivestlstrasse 17**
**D-8000 München 60 (DE)**

**EP 0 154 035 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein katalytisch wirksames Protein. Es katalysiert in Gegenwart von Sauerstoff die Oxidation von Verbindungen, die ausgewählt sind aus der Gruppe der S-Tetrahydroprotoberberine sowie aus der Gruppe der S-1-Benzylisochinoline unter Bildung der entsprechenden Protoberberine bzw. der entsprechenden 3.4-Dihydro-benzylisochinoline und Wasserstoffperoxid.

Gegenstand der Erfindung ist S-Tetrahydroprotoberberinoxidase, gekennzeichnet durch die stereospezifische Oxidation von (S)-Tetrahydroprotoberberinen bzw. (S)-1-Benzyl-1,2,3,4-tetrahydroisochinolinen zu den entsprechenden Protoberberinen bzw. 3,4-Dihydrobenzylisochinolinen. Das genannte Enzym wird durch folgende Parameter gekennzeichnet:

Molgewicht 100.000 ± 10%, bestimmt durch Gelfiltration
isoelektrischer Punkt 5,7
Temperaturoptimum 40°C
pH-Optimum pH = 8,9
$K_M$-Wert für R,S-Corypalmin bei 40°C und pH = 8,9 1,3 μMol
Intensitätsmaximum des Fluoreszenz-Emmissionsspektrums 520 nm bei einer Anregungswellenlänge von 450 nm vollständige und reversible Hemmung in Gegenwart von Acetylaceton oder Morin
Prosthetische Gruppe Flavin

S-Tetrahydroprotoberberinoxidase ist erhältlich durch Extraktion pflanzlichen Materials, das ausgewählt ist aus der Familie der Papaveracae der Berberidaceae, der Minispermaceae, der Annonaceae und der Ranunculaceae.

Beispiele für geeignetes Pflanzenmaterial aus der Familie der Papaveraceae sind Argemone-, Papaver- und Eschscholtzia- Arten. Spezielle Beispiele sind Argemone platyceras und Eschscholtzia californica.

Beispiele für Pflanzenmaterial aus der Familie der Berberidaceae sind der Berberis-Arten. Spezielle Beispiele sind Berberis beaniana, Berberis stolonifera, Berberis stolonifera, Berberis aristata und Berberis wilsonae var.

Ein spezielles Biespiel aus der Familie der Menispermaceae ist Cissampelus mucronata, sowie aus der Familie der Ranunculaceae Thalictrum glaucum.

Aus der Familie der Annonaceae sei als Beispiel Annona reticulata genannt.

Als pflanzliches Material werden vorzugsweise Zellkulturen der genannten Pflanzen herangezogen. Es können jedoch auch die differenzierten Pflanzen oder Pflanzenteile einschließlich der Wurzeln zur Gewinnung des erfindungsgemäßen Enzyms herangezogen werden.

Die Extraktion des pflanzlichen Materials wird nach Methoden durchgeführt, nach denen auch bereits bisher Enzyme aus Pflanzen extrahiert werden konnten. Üblicherweise wird zunächst so vorgegangen, daß die Zellstruktur der Pflanzen bzw. der entsprechenden Zellkulturen zerstört wird. Hierzu wird das pflanzliche Material zweckmäßigerweise eingefroren, z.B. bei der Temperatur des flüssigen Stickstoffs, ggf. zerkleinert und schließlich in einer wäßrigen Zubereitung vom pH 6 bis 10 aufgenommen. Als wäßrige Zurereitungen werden insbesondere für den genannten pH-Bereich geeignete Pufferlösungen, beispielsweise Phosphatpuffer, verwendet.

Gegebenenfalls nach Abtrennen nicht aufgeschlossenen Pflanzenmaterials erfolgt nun Proteinfällung durch Zugabe von Elektrolyt (insbesondere Ammoniumsulfat). Die gefällten Proteine werden anschließend in einer wäßrigen Pufferlösung (pH = 6 bis 10) aufgenommen und einer Gelfiltration unterworfen. Durch die Gelfiltration wird sowohl eine Entsalzung als auch eine Auftrennung des vorliegenden Proteingemisches nach Maßgabe der Molekülgröße erreicht. Zur weiteren Aufarbeitung wird derjenige Teil des Eluats aus der Gelfiltration verwendet, der die Proteine vom Molgewicht > 70.000 enthält. Die weiteren Reinigungsschritte umfassen die weitere chromatographische Auftrennung des Proteingemisches an Ionenaustauschern (z.B an Diethylaminoethylzellulose) und anschließendes Eluieren mit Kaliumchloridlösungen unterschiedlicher Konzentration. Die Auswahl an Eluat wird dabei stets nach Maßgabe der Enzymaktivitätskontrollen getroffen. Falls erwünscht, können auch andere oder zusätzliche Trennungsmethoden angewendet werden, wie beispielsweise Elektrophorese und dergleichen.

Die vorstehend beschriebenen Aufarbeitungsprozeduren werden im wäßrigen System, insbesondere in Pufferlösungen vom pH 6 bis 10, insbesondere pH 7 bis 9 durchgeführt, bei Temperaturen von 4°C bis 15°C.

Nach dem erfindungsgemäßen Verfahren werden wäßrige oder ggf. gefriergetrocknete Zubereitungen erhalten, die eine enzymatische Aktivität entsprechend einem Gehalt an S-Tetrahydroprotoberberinoxidase aufweisen.

Gegenstand der Erfindung sind somit enzymatisch aktive Zusammensetzungen, die S-Tetrahydroprotoberberinoxidase enthalten.

Das erfindungsgemäße Enzym kann oftmals mit Vorteil in immobilisiertem Zustand eingesetzt werden. Hierzu wird das Enzym in an sich bekannter Weise an Trägermaterialien physisorbiert oder chemisorbiert. Beispiele für Trägermaterialien sind Alginate, funktionelle Gruppen aufweisende Agarosen, Zellulose, Polyacrylharze (mit z.B. Oxirangruppen), Gläser, Silikate.

Das erfindungsgemäße Enzym katalysiert die Oxidation von S-Tetrahydroprotoberberinen bzw. S-1-Benzyl 1,2,3,4 Tetrahydroisochinolinen in Gegenwart von Sauerstoff, wobei die entsprechenden Protoberberine bzw. 3.4 Dihydro-benzylisochinoline gewonnen werden.

Gegenstand der Erfindung ist somit ferner ein Verfahren zum enzymatischen Oxidieren von Verbindungen der Formel

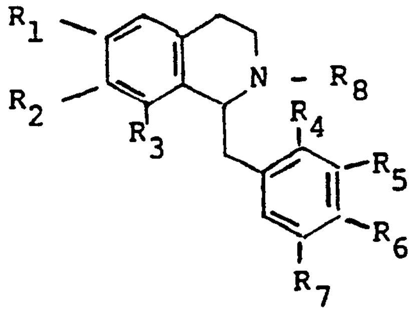

mit S-Konfiguration, wobei

$R_1$, $R_2$, $R_5$, $R_6$ und $R_7$ für Wasserstoff, Hydroxy-, Alkyl- und Alkoxy-Gruppen stehen,

$R_1 + R_2$ sowie $R_5 + R_6$ einen Dioxa-Alkylen-Ring mit 1 bis 2 Methylen-Gruppen bilden können,

$R_3$ Wasserstoff oder eine OH-Gruppe bedeutet,

$R_4$ für Wasserstoff steht

$R_8$ Wasserstoff une eine Alkylgruppe mit 1—3 C-Atomen bedeutet und

ferner $R_4 + R_8$ eine Methylen-Brücke bedeuten kann

das dadurch gekennzeichnet ist, daß die Oxidation in Gegenwart von S-Tetrahydroprotoberberinoxidase durchgeführt wird.

Vorzugsweise stehen $R_1$, $R_2$, $R_5$, und $R_6$ für Wasserstoff, OH-Gruppen, Methyl-, Ethyl-, Methoxy- und Ethoxy-Gruppen. $R_8$ ist insbesondere Wasserstoff und die Methylgruppe.

Beispiele für erfindungsgemäß zu oxidierende Verbindungen sind Corypalmin, Corydalmin, Stylopin, Isocorypalmin, Coramin, Stepholidin, Discretamin, Corytenchirin, Capaurimin, Corydalin, Retikulin, Norretikulin, Tetrahydropapaverin, N-methyl tetrahydropapaverin, Orientalin, Isoorientalin, Norisoorientalin, Nororientalin, Protosinomenin, Norprotosinomenin, Canadin, Tetrahydopalmatin, Scoulerin, Corexmin, Coclaurin; N-methyl-coclaurin; Nor-coclaurin

1-Benzyl-1,2,3,4-tetrahydro-isochinolin
1-Benzyl-6-hydroxy-1,2,3,4-tetrahydro-isochinolin
1-Benzyl-6-methoxy-1,2,3,4-tetrahydro-isochinolin
1-Benzyl-7-hydroxy-1,2,3,4-tetrahydro-isochinolin
1-Benzyl-7-methoxy-1,2,3,4-tetrahydro-isochinolin
1-(3'-Hydroxy-benzyl)-1,2,3,4-tetrahydro-isochinolin
1-(3'-Hydroxy-benzyl)-6-hydroxy-1,2,3,4-tetrahydro-isochinolin
1-(3'-Hydroxy-benzyl)-6-methoxy-1,2,3,4-tetrahydro-isochinolin
1-(3'-Hydroxy-benzyl)-7-hydroxy-1,2,3,4-tetrahydro-isochinolin
1-(3'-Hydroxy-benzyl)-7-methoxy-1,2,3,4-tetrahydro-isochinolin
1-(3',4'-Dihydroxy-benzyl)-1,2,3,4-tetrahydro-isochinolin
1-(3',4'-Dihydroxy-benzyl)-6-hydroxy-1,2,3,4-tetrahydro-isochinolin
1-(3',4'-Dihydroxy-benzyl)-6-methoxy-1,2,3,4-tetrahydro-isochinolin
1-(3',4'-Dihydroxy-benzyl)-7-hydroxy-1,2,3,4-tetrahydro-isochinolin
1-(3',4'-Dihydroxy-benzyl)-7-methoxy-1,2,3,4-tetrahydro-isochinolin
1-(3',5'-Dihydroxy-benzyl)-6-hydroxy-1,2,3,4-tetrahydro-isochinolin
1-(3',5'-Dihydroxy-benzyl)-6-methoxy-1,2,3,4-tetrahydro-isochinolin
1-(3',5'-Dihydroxy-benzyl)-7-hydroxy-1,2,3,4-tetrahydro-isochinolin
1-(3',5'-Dihydroxy-benzyl)-7-methoxy-1,2,3,4-tetrahydro-isochinolin
1-(3',4'-Dimethoxy-benzyl)-1,2,3,4-tetrahydro-isochinolin
1-(3',4'-Dimethoxy-benzyl)-6-hydroxy-1,2,3,4-tetrahydro-isochinolin
1-(3',4'-Dimethoxy-benzyl)-7-hydroxy-1,2,3,4-tetrahydro-isochinolin
1-(3',4'-Dimethoxy-benzyl)-6-methoxy-1,2,3,4-tetrahydro-isochinolin
1-(3',4'-Dimethoxy-benzyl)-7-methoxy-1,2,3,4-tetrahydro-isochinolin
1-(3'-Methoxy-4'-hydroxy-benzyl)-1,2,3,4-tetrahydro-isochinolin
1-(3'-Methoxy-4'-hydroxy-benzyl)-6-hydroxy-1,2,3,4-tetrahydro-isochinolin
1-(3'-Methoxy-4'-hydroxy-benzyl)-6-methoxy-1,2,3,4-tetrahydro-isochinolin
1-(3'-Methoxy-4'-hydroxy-benzyl)-7-hydroxy-1,2,3,4-tetrahydro-isochinolin
1-(3'-Methoxy-4'-hydroxy-benzyl)-7-methoxy-1,2,3,4-tetrahydro-isochinolin
1-(3',5'-Dimethoxy-benzyl)-1,2,3,4-tetrahydro-isochinolin
1-(3',5'-Dimethoxy-benzyl)-6-hydroxy-1,2,3,4-tetrahydro-isochinolin
1-(3',5'-Dimethoxy-benzyl)-7-hydroxy-1,2,3,4-tetrahydro-isochinolin
1-(3',5'-Dimethoxy-benzyl)-6-methoxy-1,2,3,4-tetrahydro-isochinolin
1-(3',5'-Dimethoxy-benzyl)-7-methoxy-1,2,3,4-tetrahydro-isochinolin
1-(3'-Hydroxy-4'-methoxy-benzyl)-1,2,3,4-tetrahydro-isochinolin

1-(3'-Hydroxy-4'-methoxy-benzyl)-6-hydroxy-1,2,3,4-tetrahydro-isochinolin
1-(3'-Hydroxy-4'-methoxy-benzyl)-7-hydroxy-1,2,3,4-tetrahydro-isochinolin
1-(3'-Hydroxy-4'-methoxy-benzyl)-6-hydroxy-1,2,3,4-tetrahydro-isochinolin
1-(3'-Hydroxy-4'-methoxy-benzyl)-7-hydroxy-1,2,3,4-tetrahydro-isochinolin
1-(3',4',5',-Trihydroxy-benzyl)-1,2,3,4-tetrahydro-isochinolin
1-(3',4',5',-Trihydroxy-benzyl)-6-hydroxy-1,2,3,4-tetrahydro-isochinolin
1-(3',4',5',-Trimethoxy-benzyl)-6-hydroxy-1,2,3,4-tetrahydro-isochinolin
1-(3',4',5',-Trimethoxy-benzyl)-1,2,3,4-tetrahydro-isochinolin
1-(3',4',5',-Trimethoxy-benzyl)-6-methoxy-1,2,3,4-tetrahydro-isochinolin.

Die Reaktionstemperaturen betragen 10 bis 70°C, insbesondere 35 bis 45°C. Das erfindungsgemäße Verfahren wird in Gegenwart von Sauerstoff, insbesondere in Gegenwart von Luft, durchgeführt.

Zweckmäßigerweise wird so vorgegangen, daß eine wäßrige Zubereitung von S-Tetrahydroprotoberberin bzw. S-1-Benzyl-1,2,3,4-tetrahydro-isochinolin vom pH 6 bis 10, insbesondere vom pH 8,9, die noch Lösungsvermittler wie beispielsweise Dimethylsulfoxid oder Methanol und dergleichen enthalten kann, mit einer wäßrigen Enzymzubereitung oder einer Zubereitung, in der das Enzym in immobilisiertem Zustand vorliegt, versetzt wird und unter Zutritt von Sauerstoff, insbesondere Luft, gerührt wird.

Die Menge an einzusetzendem Enzym, bezogen auf die Substratmenge, ist an sich unkritisch. Sie wird nach maßgabe des erwünschten Produktumsatzes eingestellt. Üblicherweise werden pro 1 Mol einzusetzendes Substrat Enzymmengen im Bereich von 1—50 g, inbesondere 15—20 g eingesetzt.

Der Fortschritt der Reaktion erfolgt beispielsweise durch photometrische Kontrolle.

Nach dem oben beschriebenen Verfahren werden selektiv die S-Formen der Tetrahydroprotoberberine bzw. der 1-Benzyl-1,2,3,4-tetrahydro-isochinoline oxidiert. Das Verfahren eignet sich somit zur Trennung von Enantiomerengemischen bzw. zur Gewinnung der R-Form aus der S-Form. Die R-Formen werden dabei nichtangegriffen. Sie können von den Protoberberinen bzw. 3,4-Dihydro-benzylisochinolinen, die durch Oxidation der S-Formen erhalten wurden, nach an sich bekannten Methoden, wie beispielsweise Chromatographieren, abgetrennt werden.

In einer Weiterbildung des aufgezeigten Verfahrens zur Trennung von Enantiomerengemischen von Tetrahydroprotoberberinen bzw. 1-Benzyl-1,2,3,4-tetrahydro-isochinolinen werden die oxidierten Spezien der S-Formen wieder unter Bildung der R-Form bzw. eines R/S-Gemisches zu den Ausgangsverbindungen reduziert. Es kommt auf diese Weise zur Anreicherung der R-Form. Als Reduktionsmittel kann beispielsweise Natriumborat dienen.

Vorzugsweise wird das Verfahren zur Enantiomerentrennung bzw. der Gewinnung dr R-Form aus der S-Form als Kreislaufprozeß durchgeführt. Zweckmäßigerweise wird hierbei das erfindungsgemäße Enzym in immobilisiertem Zustand eingesetzt. Ein Cyclus besteht somit, ausgehend von einem Enantiomerengemisch bzw. der S-Form, aus den folgenden Teilschritten:

a) selektive enzymatische Oxidation der S-Form unter Beibehaltung der R-Form der Ausgangsprodukte
b) Reduktion der oxidierten Produkte unter Wiedergewinnung der R-Form bzw. eines R/S-Gemisches.
c) Rückführung des R/S-Gemisches in die Oxidationsstufe

Durch mehrmalige Wiederholung dieses Kreisprozesses werden praktisch die reinen R-Formen der Tetrahydroprotoberberine bzw. der 1-Benzyl-1,2,3,4-tetrahydro-isochinolin gewonnen.

Von technischem Interesse sind die R-Formen einiger Tetrahydroprotoberberine, die u.a. als Ausgangs- bzw. Zwischenprodukte zur Herstellung physiologisch wirksamer Alkaloide eingesetzt werden.

Beispielsweise gelingt es, Enantiomerengemische von Reticulin bzw. S-Reticulin oder Enantiomerengemische von Norreticulin bzw. S-Norreticulin nach dem erfindungsgemäßen Verfahren zur Enantiomerentrennung in R-Reticulin bzw. R-Norreticulin überzuführen, die als wertvolle Zwischenprodukte zur Synthese von Codein oder Morphin dienen.

Die Erfindung wird nun anhand von Beispielen näher erläutert:

## Beispiel 1

Herstellung von S-Tetrahydroprotoberberinoxidase

220 g mit flüssigem Stickstoff eingefrorener Zellkultur aus Berberis wilsonae var. subcaulialata wurden 20 Minuten in 440 ml einer wäßrigen $KHPO_4/KH_2PO_4$-Pufferlösung (50 millimolar, pH = 7,4) gerührt. Das entstandene Zellhomogenat wurde anschließend abfiltriert und zentrifugiert. Dem Überstand wurden 236 g (bis zur 70 %igen Sättigung) festen Ammoniumsulfats zugegeben. Die dabei entstandene Proteinfällung wurde abzentrifugiert und das Zentrifugat in 20 ml $KHPO_4/KH_2PO_4$-Pufferlösung (50 millimolar, pH = 7,4) aufgenommen.

Die entstandene Lösung wurde anschließend einer Gelfiltration (an einer Ultragel AcA 44-Säule der Firma LKB) unterworfen. Die Enzymaktivität aufweisenden Fraktionen des Eluats (getestet durch die Farbreaktion von S-Norreticulin zu 1,2-Dehydronorreticulin) wurden gesammelt und einer Sepharose-Trennsäule (Matrex Blue A, Fa. Amicon) aufgegeben. Das unfraktionierte Eluat wurde anschließend an DEAE-Zellulose adsorbiert und danach mit Kaliumchloridlösung ansteigender Konzentration (von 0 bis 300 mmol KCl-Gehalt) eluiert.

Die enzymaktiven Fraktionen wurden gesammelt und durch Ultrafiltration konzentriert und entsalzt.

Das erhaltene Produkt wies eine spezifische Aktivität von 2,2 nkat/mg Protein auf. Die Ausbeute betrug 41,4%.

Das Produkt wurde noch durch diskontinuierliche Elektrophorese in Polyacrylamidgel zur Homogenität gebracht. Das verwendete Gelsystem wies im Sammelgel einen pH-Wert von 8,3, im Trenngel einen pH-Wert von 9,5 auf bei einem Gehalt von 7,5 Gew.-% Acrylamid. Der pH-Wert im Elektrodenpuffer betrug 8,3. Die Enzymaktivität aufweisenden Anteile an Trenngel wurden schließlich mit 50 millmolarer $KHPO_4$/$KH_2PO_4$-Pufferlösung eluiert.

Spezifische Enzymaktivität 6,2 nkat/mg, entsprechend einer 155-fachen Anreicherung, bezogen auf Rohextrakt.

Ausbeute: 25%.

Beispiel 2

Herstellung von immobilisierter S-Tetrahydroprotoberberinoxidase

6 ml einer Enzymzubereitung von S-Tetrahydroprotoberberinoxidase in einer wäßrigen Borsäure/Natriumborat/HCl-Pufferlösung (0,1 molar, pH = 8,9) mit einer Enzymaktivität von 2,2 nkat/mg und einem Proteingehalt von 1,12 mg/ml wurden in 50 ml einer 5 Gew.-%-igen, wäßrigen Alginatlösung unter Rühren eingetragen. Die enzymhaltige Alginatlösung wurde anschließend bei 5°C unter Rühren in 500 ml einer 0,1 molaren wäßrigen Calciumchloridlösung getropft.

Es wurde ein kugelförmiges Produkt erhalten von immobilisiertem Enzym an Calciumalginat, das noch mit Borsäure/Natriumborat/HCl-Puffer gewaschen wurde. Das Präparat wies eine Enzymaktivität von 0,55 nkat/mg Protein auf.

Beispiel 3

Oxidation von R,S-Norretikulin zu 1.2-Dehydronorretikulin

Eine wäßrige Lösung von 100 mg (316 µMol) R,S-Norretikulin in 100 ml Borsäure/Natriumborat/HCl-Pufferlösung (0,1 molar, pH = 8,9) wurde mit 10 ml einer Enzympräparation von S-Tetrahydroprotoberberinoxidase (2,1 nkat/mg; 0,15 mg Protein/ml) versetzt. Die Reaktionstemperatur betrug 37°C. Es wurde unter Luftzutritt 16 Stunden lang gerührt. Der Fortschritt der Reaktion wurde anhand von Probenentnahmen und photometrischer Analyse kontrolliert.

Nach 16 Stunden wurde eine 100 %-ige Umsetzung von S-Norretikulin zu 1,2-Dehydronorretikulin ermittelt. R-Norretikulin blieb unverändert.

Beispiel 4

Oxidation von R,S-Corypalmin zu Jatrorrhizin

100 ml einer wäßrigen Lösung von 5 mg (14,7 βMol) R,S-Corypalmin in Borsäure/Natriumborat/HCl-Puffer (pH = 8,9, 0,1 molar) wurden mit 10 ml einer Enzympräparation von S-Tetrahydroprotoberberinoxidase (2,1 nkat/mg und 0,15 mg Protein/ml) versetzt. Die Reaktionstemperatur betrug 37°C. Es wurde unter Luftzutritt 2 Stunden lang gerührt. Der Fortschritt der Reaktion wurde anhand von Probenentnahmen und photometrischen Analysen kontrolliert.

Nach 2 Stunden wurde eine 100 %-ige Umsetzung von S-Corypalmin zu Jatrorrhizin festgestellt. R-Corypalmin blieb unverändert.

Beispiel 5

Überführung von S-Norretikulin in R-Norretikulin

a) 50 g an Calciumalginat immobilisierter S-Tetrahydroprotoberberinoxidase (in Perlform) analog Beispiel 2 mit einer Enzymaktivität von 0,2 nkat wurden in einer Lösung von 30 mg S-Norretikulin in 100 ml wäßriger Borsäure/Natriumborat/HCl-Pufferlösung (0,1 molar, pH = 8,9) bei einer Temperatur von 30°C unter Luftzutritt 150 Stunden geschüttelt. Es wurde anhand von photometrischen Analysen eine vollständige Umsetzung von S-Norretikulin in 1,2-Dehydronorretikulin festgestellt.

Danach wurde abfiltriert und das Filtrat portionsweise mit 20 mg Natriumboranat versetzt. Die ursprünglich gelbe Lösung wurde augenblicklich entfärbt. Es wurde eine 1;1-Gemisch von S-Norretikulin und R-Norretikulin erhalten.

b) Das gemäß a) erhaltene S/R-Norretikulin-Gemisch wurde durch Zugabe von 0,1 normaler Salzsäure auf einen pH von 8,9 gebracht und nach der gemäß a) beschriebenen Arbeitsweise der enzymatischen Oxidation unterworfen.

Es wurde nach Reduktion ein Gemisch erhalten, das 3 Teile R-Norretikulin und 1 Teil S-Norretikulin enthielt.

Beispiel 6

Oxydation von R,S-Canadin zu Berberin

Es wurde die Arbietsweise gemäß Biespiel 4 wiederholt mit der Abänderung, daß anstatt R,S-Corypalmin 5 g (14,7 mMol) R,S-Canadin eingesetzt wurden.

Nach 2 Stunden wurde eine 100 %ige Umsetzung von S-Canadin zu Berberin festgestellt. R-Canadin blieb unverändert.

Beispiel 7

Oxidation von R,S-[1-(3',5'-Dihydroxy-benzyl)-6-methoxy-1,2,3,4-tetrahydro-isochinolin]

Es wurde die Arbeitsweise gemäß Beispiel 3 wiederholt mit der Abänderung, daß anstatt R,S-Norretikulin 100 mg R,S-[1-(3',5'-Dihydroxy-benzyl)-6-methoxy-1,2,3,4-tetrahydro-isochinolin] eingesetzt wurden.

Nach 16 Stunden wurde eine 100 %-ige Umsetzung von S-[1-(3',5'-Dihydroxy-benzyl)-6-methoxy-1,2,3,4-tetrahydro-isochinolin] zu 1-(3',5'-Dihydroxy-benzyl)-6-methoxy-3,4-dihydroisochinolin festgestellt. Die R-Form blieb unverändert.

**Patentansprüche für die Vertragsstaaten: CH DE LI SE**

1. S-Tetrahydroprotoberberinoxidase, gekennzeichnet durch die stereospezifische Oxidation von (S)-Tetrahydroprotoberberinen bzw. (S)-1-Benzyl-1,2,3,4-tetrahydroisochinolinen zu den entsprechenden Protoberberinen bzw. 3,4-Dihydrobenzylisochinolinen.

2. S-Tetrahydroprotoberberinoxidase gemäß Anspruch 1, gekennzeichnet durch die folgenden Parameter

a) Molgewicht 100.000 ± 10%, bestimmt durch Gelfiltration

b) isoelektrischer Punkt 5,7

c) Temperaturoptimum 40°C

d) pH-Optimum pH = 8,9

e) $K_M$-Wert für R,S-Corypalmin bei 40°C und pH = 8,9 1,3 µMol

f) Intensitätsmaximum des Fluoreszenz-Emmissionsspektrums 520 nm einer Anregungswellenlänge von 450 nm

g) vollständige und reversible Hemmung in Gegenwart von Acetylaceton oder Morin

h) Prosthetische Gruppe Flavin.

3. S-Tetrahydroprotoberberinoxidase gemäß Anspruch 1, erhältlich durch Extraktion aus Pflanzenmaterial aus der Familie der Papaveraceae, der Berberidaceae, der Menispermaceae, der Annonaceae und der Ranunculaceae.

4. Enzymatisch aktive Zusammensetzungen, die S-Tetrahydroprotoberberinoxidase gemäß Anspruch 1 enthalten.

5. Verfahren zur Herstellung von S-Tetrahydroprotoberberinoxidase gemäß Anspruch, dadurch gekennzeichnet, daß Pflanzenmaterial aus der Familie der Papaveraceae, der Berberidaceae, der Menispermaceae, der Annonaceae und der Ranunculaceae extrahiert wird.

6. Verfahren zum enzymatischen Oxidieren von Verbindungen der allgemeinen Formel

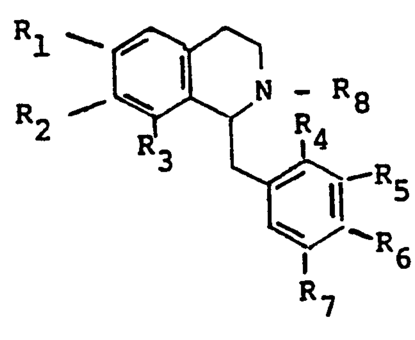

mit S-Konfiguration, wobei

$R_1$, $R_2$, $R_5$, $R_6$ und $R_7$ für Wasserstoff, Hydroxy-, Alkyl- und Alkoxy-Gruppen stehen,

$R_1$ + $R_2$ sowie $R_5$ + $R_6$ einen Dioxa-Alkylen-Ring mit 1 bis 2, Methylen-Gruppen bilden können,

$R_3$ Wasserstoff oder eine OH-Gruppe bedeutet,

$R_4$ für Wasserstoff steht

$R_8$ Wasserstoff une eine Alkylgruppe mit 1—3 C-Atomen bedeutet und

ferner $R_4$ + $R_8$ eine Methylen-Brücke bedeuten kann

dadurch gekennzeichnet, daß die Oxidation in Gegenwart von S-Tetrahydroprotoberberinoxidase gemäß Anspruch 1 durchgeführt wird.

**Patentansprüche für den Vertragsstaat: AT**

1. Enzymatisch aktive Zusammensetzungen, die S-Tetrahydroprotoberberinoxidase gekennzeichnet durch die stereospezifische Oxidation von (S)-Tetrahydroprotoberberinen bzw. (S)-1-Benzyl-1,2,3,4-tetrahydroisochinolinen zu den entsprechenden Protoberberinen bzw. 3,4-Dihydrobenzylisochinolinen enthalten.

2. Verfahren zur Herstellung on S-Tetrahydroprotoberberinoxidase, gekennzeichnet durch die

stereospezifische Oxidation von (S)-Tetrahydroprotoberberinen bzw. (S)-1-Benzyl-1,2,3,4-tetrahydroisochinolinen zu den entsprechenden Protoberberinen bzw. 3,4-Dihydrobenzylisochinolinen dadurch gekennzeichnet, daß Pflanzenmaterial aus der Familie der Papaveraceae, der Berberidaceae, der Menispermaceae, der Annonaceae und der Ranunculaceae extrahiert wird.

3. Verfahren zum enzymatischen Oxidieren von Verbindungen der allgemeinen Formel

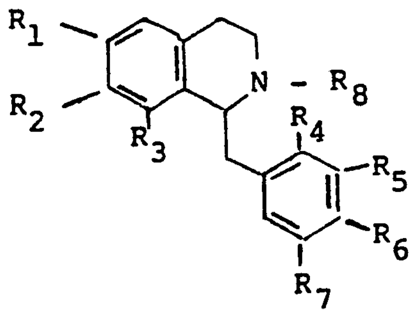

mit S-Konfiguration, wobei

$R_1$, $R_2$, $R_5$, $R_6$ und $R_7$ für Wasserstoff, Hydroxy-, Alkyl- und Alkoxy-Gruppen stehen,

$R_1 + R_2$ sowie $R_5 + R_6$ einen Dioxa-Alkylen-Ring mit 1 bis 2, Methylen-Gruppen bilden können,

$R_3$ Wasserstoff oder eine OH-Gruppe bedeutet,

$R_4$ für Wasserstoff steht

$R_8$ Wasserstoff une eine Alkylgruppe mit 1—3 C-Atomen bedeutet und

ferner $R_4 + R_8$ eine Methylen-Brücke bedeuten kann

dadurch gekennzeichnet, daß die Oxidation in Gegenwart von S-Tetrahydroprotoberberinoxidase gekennzeichnet durch die stereospezifische Oxidation von (S)-Tetrahydroprotoberberinen bzw. (S)-1-Benzyl-1,2,3,4-tetrahydroisochinolinen zu den entsprechenden Protoberberinen bzw. 3,3-Dihydrobenzylisochinolinen durchgeführt wird.

**Revendications pour les Etats contractants: CH DE LI SE**

1. Tétrahydro-protoberbérine-(S)-oxydase caractérisée par l'oxydation stéréospécifique de tétrahydro-protoberbérines-(S) ou de benzyl-1 tétrahydro-1,2,3,4 isoquinoléines-(S) en les protoberbérines et dihydro-3,4 benzylisoquinoléines correspondantes.

2. Tétrahydro-protoberbérine-(S)-oxydase selon la revendication 1, caractérisée par les paramètres suivants:

a) Masse moléculaire: 100.000 ± 10%, déterminée par filtration sur gel,

b) Point iso-électrique: 5,7,

c) Optimum de température: 40°C,

d) Optimum de pH = 8,9,

e) Valeur de $C_M$ pour la corypalmine-(R,S) à 40°C et à une pH de 8,9:1,3 μmol,

f) Maximum d'intensité du spectre d'émission de fluorescence à 520 nm pour une longueur d'onde d'excitation de 450 nm,

g) Inhibition totale et réversible en présence d'acétylacétone ou de morine,

h) Groupe prosthétique:flavine.

3. Tétrahydro-protoberbérine-(S)-oxydase selon la revendication 1, qui peut être obtenue par extraction d'une matière végétale des familles des papavéracées, des berbéridacées, des ménispermacées, des annonacées et des renonculacées.

4. Compositions douées d'une activité enzymatique qui contiennent de la tétrahydro-protoberbérine-(S)-oxydase selon la revendication 1.

5. Procédé de préparation de la tétrahydro-protoberbérine-(S)-oxydase selon la revendication 1, procédé caractérisé en ce qu'on extrait une matière végétale des familles des papavéracées, des berbéridacées, des ménispermacées, des annonacées et des renonculacées.

6. Procédé pour oxyder par voie enzymatique des composés à configuration S répondant à la formule générale suivante:

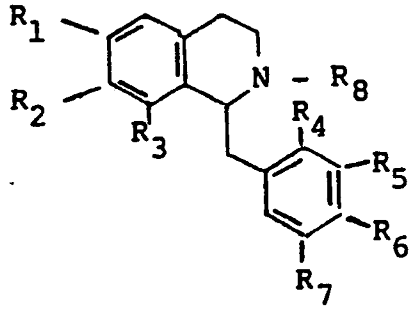

dans laquelle

$R_1$, $R_2$, $R_5$, $R_6$ et $R_7$ représentent chacun l'hydrogène, un hydroxy, un alkyle ou un alcoxy,

$R_1$ + $R_2$ ainsi $R_5$ + $R_6$ peuvent former un cycle dioxa-alkylène contenant un ou deux radicaux méthylènes,

$R_3$ représente l'hydrogène ou un radical —OH,

$R_4$ représente l'hydrogène,

$R_8$ représente l'hydrogène ou un alkyle contenant de 1 à 3 atomes de carbone et

$R_4$ + $R_8$ peut représenter un pont méthylène, procédé caractérisé en ce qu'on effectue l'oxydation en présence de la tétrahydro-protoberbérine-(S)-oxydase selon la revendication 1.

**Revendications pour l'Etat contractant: AT**

1. Compositions douées d'une activité enzymatique qui contiennent de la tétrahydro-protoberbérine-(S)-oxydase caractérisée par l'oxydation stéréospécifique de tétrahydro-protoberbérines-(S) ou de benzyl-1 tétrahydro-1,2,3,4 isoquinoléines-(S) en les protoberbérines ou dihydro-3,4 benzylisoquinoléines correspondantes.

2. Procédé de préparation de la tétrahydro-protoberbérine-(S)-oxydase caractérisé par l'oxydation stéréospécifique de tétrahydro-protoberbérine-(S) ou de benzyl-1 tétrahydro-1,2,3,4 isoquinoléines-(S) en les protoberbérines ou dihydro-3,4 benzylisoquinoléines correspondantes, procédé caractérisé en ce qu'on extrait de la matière végétale des familles des papavéracées, des berbéridacées, des ménispermacées, des annonacées et des renonculacées.

3. Procédé pour oxyder par voie enzymatique des composés à configuration S repondant à la formule générale suivante:

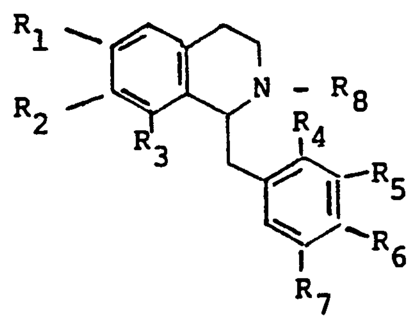

dans laquelle

$R_1$, $R_2$, $R_5$, $R_6$ et $R_7$ représentent chacun l'hydrogène, un hydroxy, un alkyle ou un alcoxy,

$R_1$ + $R_2$ ainsi $R_5$ + $R_6$ peuvent former un cycle dioxa-alkylène contenant un ou deux radicaux méthylènes,

$R_3$ représente l'hydrogène ou un radical —OH,

$R_4$ représente l'hydrogène,

$R_8$ représente l'hydrogène ou un alkyle contenant de 1 à 3 atomes de carbone et

$R_4$ + $R_8$ peut représenter un pont méthylène, procédé caractérisé en ce qu'on effectue l'oxydation en présence de la tétrahydro-protoberbérine-(S)-oxydase caractérisée par l'oxydation stéréospécifique de tétrahydro-protoberbérines-(S) ou de benzyl-1 tétrahydro-1,2,3,4 isoquinoléines-(S) en les protoberbérines ou dihydro-3,4-benzylisoquinoléines correspondantes.

**Claims for the Contracting States: CH DE LI SE**

1. S-Tetrahydroprotoberberine oxidase, characterized by the stereospecific oxidation of (S)-tetrahydroprotoberberines and (S)-1-benzyl-1,2,3,4-tetrahydroisoquinolines to the corresponding protoberberines and 3,4-dihydrobenzylisoquinolines respectively.

2. S-Tetrahydroprotoberberine oxidase according to Claim 1, characterized by the following parameters

a) molecular weight 100,000 ± 10% determined by gel filtration

b) isoelectric point 5,7

c) temperature optimum 40°C

d) pH optimum pH = 8,9

e) $K_M$ value for R,S-corypalmine at 40°C and pH = 8,9 1,3 µmol

f) intensity maximum in the fluorescence emission spectrum 520 nm with an excitation wavelength of 450 nm

g) complete and reversible inhibition in the presence of acetylacetone or morin

h) prosthetic group flavin.

3. S-Tetrahydroprotoberberine oxidase according to Claim 1, obtainable by extraction of plant material from the family of Papaveraceae, of Berberidaceae, of Menispermaceae, of Annonaceae and of Ranunculaceae.

4. Compositions which have enzymatic activity and contain S-tetrahydroprotoberberine oxidase according to Claim 1.

5. Process for the preparation of S-tetrahydroprotoberberine oxidase according to claim 1, characterized in that plant material from the family of Papaveraceae, of Berberidaceae, of Menispermaceae, of Annonaceae and of Ranunculaceae is extracted.

6. Process for the enzymatic oxidation of compounds of the general formula

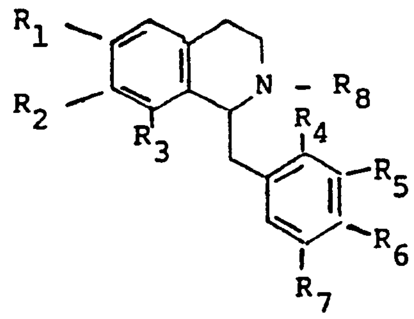

with the S configuration, where

$R_1$, $R_2$, $R_5$, $R_6$ and $R_7$ stand for hydrogen, hydroxyl, alkyl and alcoxy groups,

$R_1 + R_2$ and $R_5 + R_6$ form a dioxa-alkylene ring with 1 to 2 methylene groups,

$R_3$ denotes hydrogen or an OH group,

$R_4$ stands for hydrogen,

$R_8$ denotes hydrogen and an alkyl group with 1—3 C atoms, and, furthermore,

$R_4 + R_8$ can denote a methylene bridge, characterized in that the oxidation is carried out in the presence of S-tetrahydroprotoberberine oxidase according to Claim 1.

**Claims for the Contracting State: AT**

1. Compositions which have enzymatic activity and contain S-tetrahydroprotoberberine oxidase characterized by the stereospecific oxidation of (S)-tetrahydroprotoberberines and (S)-1-benzyl-1,2,3,4-tetrahydroisoquinolines to the corresponding protoberberines and 3,4-dihydrobenzylisoquinolines respectively.

2. Process for the preparation of S-tetrahydroprotoberberine oxidase, characterized by the stereospecific odixation of (S)-tetrahydroprotoberberines and (S)-1-benzyl-1,2,3,4-tetrahydroisoquinolines to the corresponding protoberberines and 3,4-dihydrobenzylisoquinolines respectively, characterized in that plant material from the family of Papaveraceae, of Berberidaceae, of Menispermaceae, of Annonaceae and of Ranunculaceae is extracted.

3. Process for the enzymatic oxidation of compounds of the general formula

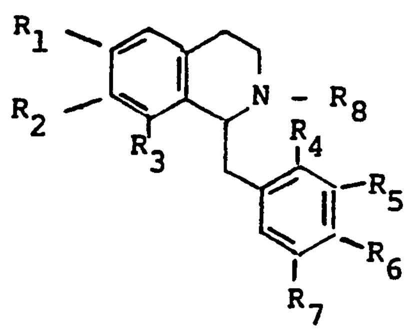

with the S configuration, where

$R_1$, $R_2$, $R_5$, $R_6$ and $R_7$ stand for hydrogen, hydroxyl, alkyl and alkoxy groups,

$R_1 + R_2$ and $R_5 + R_6$ form a dioxa-alkylene ring with 1 to 2 methylene groups,

$R_3$ denotes hydrogen or an OH group,

$R_4$ stands for hydrogen,

$R_8$ denotes hydrogen and an alkyl group with 1—3 C atoms, and, furthermore,

$R_4 + R_8$ can denote a methylene bridge, characterized in that the oxidation is carried out in the presence of S-tetrahydroprotoberberine oxidase characterized by the stereospecific oxidation of (S)-tetrahydroprotoberberines and (S)-1-benzyl-1,2,3,4-tetrahydroisoquinolines to the corresponding protoberberines and 3,4-dihydrobenzylisoquinolines respectively.